# EUROPEAN PATENT APPLICATION

(11) **EP 1 353 178 A1**
(43) Date of publication of application: **15.10.2003**
(21) Application number: 01271864.9
(22) Date of filing: 21.12.2001
(51) Int. Cl.: G01N 33/50, C12Q 1/68, G01N 33/53, C12M 1/00, C12N 15/09

(54) **HIGH-THROUGHPUT SCREENING SYSTEM BY MICROARRAY**

(30) Priority: 22.12.2000 JP 2000391709
(71) Applicant: CHUGAI SEIYAKU KABUSHIKI KAISHA, Tokyo, 115-8543 (JP)
(72) Inventor: JONES, Michael H., CHUGAI SEIYAKU KABUSHIKI KAISHA, Shizuoka 412-8513 (JP)
(74) Representative: Harding, Charles Thomas
(86) International application number: JP0111288
(87) International publication number: WO02052261

(57) **Abstract**

The present inventors determined that the expression of a disease gene can be monitored using as an indicator the expression of genes (cluster genes) that are identified as a cluster located downstream of the disease-related gene. Analysis of the expression of the disease gene via microarrays using the expression of the cluster genes as an indicator enables efficient and exhaustive screening of candidate compounds for the treatment of the disease.

## Description

### Technical Field

The present invention relates to a method of estimating a candidate compound for the treatment of a specific disease and a method of screening for such compound.

### Background Art

Simple assays have been developed for typical high-throughput screening (HTS) methods that are used for the identification of therapeutic compounds for specific diseases. For example, if an interaction of any two proteins is identified as one of the pathophysiological processes of a specific disease, an assay method can be developed to identify drugs that specifically block the interaction. However, this assay method is based on the principle in which genes encoding the two proteins are artificially introduced to detect a specific event between the proteins using an artificial reporter assay. Thus, this method has a disadvantage that it is unclear whether the isolated compound indeed has a biological effect on cells of natural state.

In addition, although it is possible to carry out screening using cells of natural state in normal experimental scale, such a method has a limitation in the number of samples, in particular the number of genes by high-throughput screening at a time.

Therefore, development of an effective method of identifying and screening for compounds for treating diseases where compounds are effective in cells in their natural state has been desired in the art.

### Disclosure of the Invention

The present invention was made in view of the above observations. Its objective is to provide a novel method of estimating and screening for a candidate compound for treating a disease where the compound is expected to have a biological effect in natural cells.

The expression of thousands of genes can be analyzed in parallel in a single assay employing microarrays. Through further analysis based on the data from such experiments, a group of co-regulated genes can be categorized and identified as a cluster. Studies using yeast have demonstrated that mutations generating such clusters occur with high frequency in genes located in the pathway upstream of the cluster (Timothy R. et al., Functional Discovery via a Compendium of Expression Profiles. Cell 102 (1):109-126 (2000)). The present inventors focused on this fact and hypothesized that the expression of a disease-related gene (disease gene) can be monitored using as an indicator of the expression of a group of genes (cluster genes) identified as a cluster located downstream of the disease gene. Specifically, the present inventors contemplated that the expression of the cluster gene can be utilized as a reporter for the expression of the disease gene.

Accordingly, the present inventors carried out extensive studies to apply the above concept to screening for candidate compounds for the treatment of diseases. As a result, the present inventors succeeded in developing a method for effectively identifying and screening candidate compounds for disease treatment by comprehensively detecting the expressions of cluster genes on microarrays as a natural indicator of the expression of a disease gene.

In the method of the present invention, firstly, oligonucleotides that hybridize with multiple genes constituting a cluster related to a specific disease are immobilized on a platform. Next, cDNAs are prepared from a cell related to the disease which cell is treated with a test compound. Then, the prepared cDNAs are contacted with the oligonucleotides immobilized on the platform. Following the contact, the expression of the cluster genes in the cell is monitored. Then, a compound that can restore the expression levels of cluster genes to those found in normal cell is selected from the test compounds. Thus, a candidate compound can be obtained for the treatment of the disease. The cluster genes used as the reporter in the present invention exist in a cell in their natural state, and therefore, directly reflects the effect of the compound on the cell. Thus, a compound obtained by the above method is expected to produce an actual effect in the treatment of the disease.

Thus, this invention relates to a novel method of estimating and screening for a candidate compound for the treatment of a disease where such a compound can be expected to show a biological effect on cells. More specifically, it provides:
(1) a method for estimating whether a test compound can be a candidate for treating a specific disease, which method comprises the steps of:
   (a) providing
      (i) cDNAs derived from a disease cell treated with a test compound, and
      (ii) a platform immobilized with nucleotide probes that respectively hybridize with multiple cluster genes constituting a cluster related to the disease,
   (b) contacting the cDNAs of (a) (i) with the platform of (a) ( ii) ,
   (c) measuring the expression levels of the cluster genes in the disease cell treated with the test compound by detecting cDNAs that hybridized with the nucleotide probes immobilized on the platform, and
   (d) estimating whether the expression levels of the cluster genes in the disease cell treated with the test compound are restored to that in normal cell;
(2) a method of screening for candidate compounds for the treatment of a specific disease, which method comprises the steps of:
   (a) providing
      (i) cDNAs derived from a disease cell treated with a test compound, and
      (ii) a platform immobilized with nucleotide probes that respectively hybridize with multiple cluster genes constituting a cluster related to the disease,
   (b) contacting the cDNAs of (a) (i) with the platform of (a) ( ii) ,
   (c) measuring the expression levels of the genes constituting the cluster in the disease cell treated with the test compound by detecting cDNAs that hybridized with the nucleotide probes immobilized on the platform,
   (d) estimating whether the expression levels of the cluster genes in the disease cell treated with the test compound are restored to that in normal cell, and
   (e) selecting the test compound that restored the expression levels of the genes constituting the cluster in the disease cell treated with the compound to that in control cell; and,
(3) a candidate compound for the treatment of a specific disease isolated by the method according to (2).

The present invention provides a method of estimating whether a test compound can serve as a candidate for the treatment of a specific disease.

According to the present invention, firstly, (i) cDNAs derived from a disease cell treated with a test compound, and (ii) a platform immobilized with nucleotide probes that respectively hybridize with multiple genes that constitute a cluster related to a disease are provided (step (a)).

Herein, the term "disease cell" refers to cells derived from a diseased organism. Preferably, it is a cell from the diseased tissue. The "disease cell" of the present invention also. includes any established cell line representing a disease (for example, tumor cell lines for tumors).

Herein, the diseased organism is not restricted in any way. For screening a candidate compound as a therapeutic agent for a human disease, the diseased organism is preferably a mammal (for example, a mammalian model for a human disease, such as rats and mice), and most preferably humans. For example, diseases to be treated and preferable disease cells include the following:
Tumors: tumor tissues, and tumor cell lines;
Proliferative disorders: cell lines derived from diseases such as prostatomegaly, hysteromyoma, and endometriosis; and cells from animal models or patients of such diseases; and
Circulatory diseases: cell lines derived from diseases such as hypertension, arteriosclerosis, cardiac infarction, and coronary restenosis ; and cells from animal models or patients of such diseases. Furthermore, other diseases such as diabetes, inflammatory diseases, immune disorders, and infectious diseases are also included.

Herein, the phrase "disease cell treated with a test compound" refers to cells directly contacted with the compound and those derived from an animal injected with the test compound. For example, when the disease cell is derived from a mammalian model of a human disease, a cell derived from the disease model administered with a test compound is also included within the scope of the phrase "disease cell treated with a test compound" of the present invention.

For example, when a disease cell is an isolated cell, the cell may be treated with a test compound by the method described by Scherf et al. (Scherf U. , Ross D.T., Waltham M. , Smith L.H. , Lee J.K., Tanabe L., Kohn K.W., Reinhold W.C., Myers T.G., Andrews D.T., ScudieroD A., Eisen M.B., Sausville E.A., Pommier Y., Botstein D., Brown P.O., and Weinstein J.N., A gene expression database for the molecular pharmacology of cancer. Nat. Genet. 24: 236-244 (2000)). When an animal is used, the compound can be orally or parenterally administered.

Any compound can be used as a test compound for the screening of this invention. For example, it includes cell extracts, cell culture supernatants, products of fermentative microorganisms, extracts of marine organisms, plant extracts, purified proteins or crudely-purified proteins, peptides, non-peptidic compounds, synthetic low molecular weight compounds and natural compounds.

A cDNA can be prepared from a disease cell by methods known to those skilled in the art. According to a preferable embodiment of cDNA preparation, first the total RNA is extracted from a disease cell. The preparation of total RNA can be performed by methods known to those skilled in the art, for example, as follows. In general, any known method or kit can be used for the preparation as long as total RNA of high purity can be prepared. For example, "RNA later" (Ambion) is used for pretreatment, and then total RNA is extracted with Isogen (Nippon Gene) basically according to the accompanying protocols. Specifically, harvested cells are washed with PBS, resuspended in "RNA later", and stored at 4°C for at least 1 hr. After centrifugation at 15K x *g* for 5 min, the supernatant is discarded, and the pellet is dispersed. Isogen (1 ml/2x 10⁶ cells) is added to the pellet, and completely mixed therewith. Then, this mixture is, left standing at room temperature for 5 min, and extracted twice with chloroform. Then, the extract is treated with isopropanol, washed with 80% ethanol, dried, and suspended/dissolved in RNase-free water.

Next, the extracted total RNA is used as a template for cDNA synthesis using reverse transcriptase to prepare a cDNA sample. The cDNA synthesis from the total RNA can be performed according to methods known to those skilled in the art.

If necessary, the prepared cDNA sample can be labeled for detection. Any labeling agent can be used as long as it enables detection and includes, for example, fluorescent substances and radioisotopes, etc.. The labeling can be performed according to general methods used by those skilled in the art (Luo L. et al., Gene expression profiles of laser-captured adjacent neuronal subtypes. Nat. Med. 5(1): 117-122 (1999)).

For example, cDNA synthesis and fluorescence labeling can be carried out as follows. Basically, the labeling is conducted using a fluorescent-labeled dUTP in the reverse transcription reaction. For example, 10 µg of total RNA is mixed with 2 µl of random hexamer (2 µg/ml) , 2 µl of oligo-dT primer (0.5 µg/ml), and.RNase free-H₂O to a total volume of 10 µl; and heated at 70°C for 10 min. After cooling on ice for 1 min, keeping the mixture cold 15 µl of labeling mix, 2.5 µl of Cy dUTP (AP Biotech) , 1 µl of RNaseOUT (Life Tech), and 2.0 µl of Superscript II reverse transcriptase (Life Tech) is immediately mixed; and then incubated at 42°C for 2 hr. After the incubation, the reaction mixture is cooled on ice, mixed with 20 µl of 5x second strand synthesis buffer, 50 µl of H₂O, and 1 µl of *E. coli* DNA ligase; incubated at 16°C for 1 hr; and then the reaction is immediately stopped by adding 4.5 µl of 25 mM EDTA (pH 8.0) . 5 µl of 1.0 M NaOH is added and mixed to the reaction mixture, the mixture is heated at 70°C for 10 min, and then neutralized by the addition of 5 µl of 1.0 M HCl.

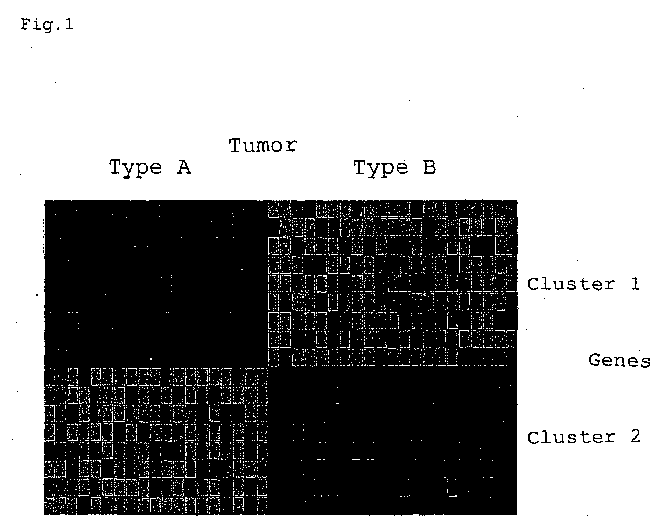

Herein, a "cluster" is defined as a group of genes belonging to a common downstream pathway in the functional mechanism for a gene related to a specific disease (disease gene). For example, the identification of the cluster genes can be carried out by the following method. The expression profiles of many different samples are compared and analyzed in a series of assays wherein the same control sample is used in respective experiments. The two-dimensional clustering method is usually used to order the expression profile of each gene to the multiple samples (Iyer VR, Eisen MB, Ross DT, Schuler G, Moore T, Lee JC, Trent JM, Staudt LM, Hudson J Jr, Boguski MS, Lashkari D, Shalon D, Botstein D, Brown PO. , The transcriptional program in the response of human fibroblasts to serum. Science 283 (5398):83-87 (1999); Golub TR, Slonim DK, Tamayo P, Huard C, Gaasenbeek M, Mesirov JP, Coller H, Loh ML, Dowing JR, Caligiuri MA, Bloomfield CD, Lander ES., Molecular classification of cancer; class discovery and class prediction by gene expression monitoring. Science 286 (5439):531-537 (1999); Hughes TR, Marton MJ, Jones AR, Roberts CJ, Stoughton R, Armour CD, Bennett HA, Coffey E, Dai H, He YD, Kidd MJ, King AM, Meyer MR, Slade D, Lum PY, Stepaniants SB, Shoemaker DD, Gachotte D, Chakraburtty K, Simon J, BardM, FriendSH., Functional discovery via a compendium of expression profiles. Cell 102 (1) :109-126 (2000)). Fig. 1 shows a simplified typical output data obtained by the clustering technique. These clustering methods are hierarchical, and thus samples exhibiting the most similar gene expression profiles are aligned on a single axis while genes exhibiting the most similar expression profiles to a sample are aligned on another axis. Such a group of genes aligned on a single axis is identified as a cluster gene.

The number of the genes identified as a cluster gene of the present invention is favorably multiple so that the genes can function as a reporter. Generally, the more the number of genes identified as the cluster gene, the higher the accuracy of the cluster gene as a reporter gene will be. The minimal number of genes required for separating them as a cluster to use as a strong reporter can be determined experimentally and empirically for each cluster, but the number is normally ten or more, preferably 20 or more, and more preferably 50 or more genes. Generally, 10 to 20 genes are assumed to be sufficient as a cluster gene. Some genes are already identified as a cluster for cancer (Alizadeh AA, Eisen MB, Davis RE, Ma C, Lossos IS, Rosenwald A, Boldrick JC, Sabet H, Tran T, Yu X, Powell JI, Yang L, Marti GE, Moore T, Hudson J Jr, Lu L, Lewis DB, Tibshirani R, Sherlock G, Chan WC, Greiner TC, Weisenburger DD, Armitage JO, Warnke R, Levy R, Wilson W, Grever MR, Byrd JC, Botstein D, Brown PO, Staudt LM., Distinct types of diffuse large B-cell lymphoma identified by gene expression profiling. Nature 403 (6769):503-511 (2000); Perou CM, Sorlie T, Eisen MB, van de Rijn M, Jeffrey SS, Rees CA, Pollack JR, Ross DT, Johnsen H, Akslen LA, Fluge O, Pergamenschikov A, Williams C, Zhu SX, Lonning PE, Borresen-Dale AL, Brown PO, Botstein D., Molecular portraits of human breast tumors. Nature 406 (6797) :747-752 (2000)). More cancers are currently being analyzed, and thus presently known clusters may be reconfirmed, or novel cluster of genes may be identified or discovered. Most of the known clusters include 10 or more genes, and are thus expected to function as strong reporters in the present invention.

Herein, the term "platform" refers to a plate of material on which a nucleotide can be immobilized. Nucleotides of the present invention include oligonucleotides and polynucleotides. Any platform can be used in the present invention as long as nucleotides can be immobilized thereon. Generally, platforms used in microarray techniques can be preferably used. The nomenclature used in the field of microarray is slightly different from that used in conventional hybridization techniques such as southern blotting. In microarray, a DNA immobilized on a slide glass is called the probe, and a labeled DNA in solution is called the target (see, Phimster B., Going Global. Nature Genetics 21 (Supplement):1 (1999)). Thus, the above nucleotide immobilized on a platform may be referred herein to as a nucleotide probe.

The advantage of the microarray technique is that it allows an extremely complicated target comprising cDNA from total RNA of cells to hybridize with immobilized nucleotide probes using a very small volume of hybridization solution. Microarrays are generally composed of thousands of nucleotides printed on a platform at a high density. Generally, these DNAs are printed on the surface layer of a non-porous platform. Typical surface layer of the platform is glass; however, porous membranes like nitrocellulose membranes may also be used. There are two types of immobilized nucleotides (microarrays): one is the microarray developed by Affymetrix, which is based on oligonucleotides while the other is a cDNA microarray mainly developed by Stanford University. In the oligonucleotide array, the oligonucleotides are normally synthesized *in situ.* For example, *in situ* oligonucleotide synthesis, such as those utilizing the photolithographic technique (Affymetrix) and the ink jet technique to immobilize a chemical compound (Rosetta Inpharmatics), are known in the art. Any of these methods can be used in the preparation of a platform for the present invention.

Any nucleotide probe can be immobilized onto a platform as long as it can specifically hybridize with a cluster gene. The nucleotide probes of the present invention include oligonucleotides and cDNAs. Herein, the phrase "specifically hybridize" indicates that the probe substantially hybridizes with the cluster gene, but not with other genes. The probe does not have to be completely complementary to the nucleotide sequence of the cluster gene as long as it specifically hybridizes with the cluster gene.

The number of the kinds of nucleotide probes that should be immobilized onto a platform is determined depending on the cluster genes whose expressions are desired to be detected in this invention. For example, when ten cluster genes are selected, nucleotide probes that can specifically hybridize with these ten genes, respectively, are immobilized on the platform. The nucleotide probe corresponding to the respective cluster gene is not necessarily limited to a single kind of nucleotide, and may be a mixture of multiple kinds of nucleotide probes that are complementary to arbitrary regions of the cluster gene whose expression is desired to be detected.

The length of a nucleotide probe to be immobilized on a platform is typically 100 to 4000 bases, preferably 200 to 4000 bases, and more preferably 500 to 4000 bases when cDNA is immobilized. When oligonucleotides are immobilized, the length thereof is typically 15 to 500 bases, preferably 30 to 200 bases, and more preferably 50 to 200 bases.

In a preferred embodiment of the present invention, candidate compounds for treating multiple diseases may be screened. In this case, multiple cluster genes selected from respective clusters associated with multiple diseases are immobilized onto a platform.

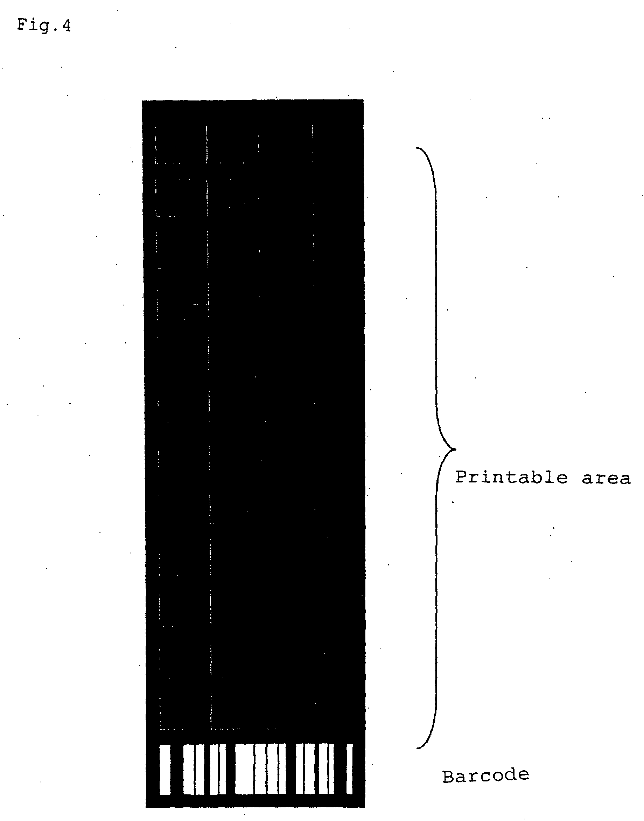

The step of immobilizing an oligonucleotide onto a platform is generally called "printing." Specifically, for example, the oligonucleotide can be printed as follows, but the method is not limited thereto. Several kinds of oligonucleotide probes are printed within an area of 4.5 mm x 4.5 mm. A single pin can be used to print each array, and thus, 48 repeated arrays can be printed on a standard microscopy slide using a tool with 48 pins (Fig. 4) . This means that 48 different compounds can be assayed on a single slide.

In the next step of the present invention, the cDNA of step (a) (i) is contacted with the platform of (a)(ii) (step (b)).

In this step, the cDNA samples are hybridized to the nucleotide probes on the platform, which probes can specifically hybridize with the cluster genes. The composition of the hybridization solution and the conditions for hybridization varies according to factors, such as the length of the nucleotide probes on the platform; however, the hybridization can be conducted according to standard methods known to those skilled in the art. Specifically, the following conditions may be used for the hybridization.
Buffer: 5x SSC, 1% SDS, 50% formamide;
Temperature: 42°C;
Incubation time: 12-16 hr; and
Stringency of washing: 1x SSC, 42°C for 10 min.

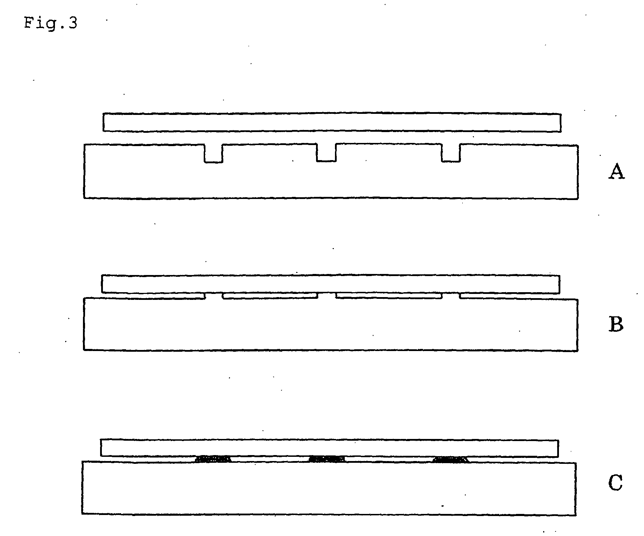

In order to independently perform multiple assays on a single platform, a physical barrier is preferably formed to prevent contamination of nearby samples during hybridization. For example, separated areas can be formed by physically partitioning the platform itself or the surface material on the platform. Specifically, the following three methods can be used (Fig. 3) . According to the first method, grooves are cut in the slide (Fig. 3A). A groove of enough depth and volume allows an excess amount of hybridization buffer to be drained and held therein without the leaking of the buffer into the surrounding areas to avoid contamination. The second method utilizes a seal made of rubber or plastic for separating each array (Fig. 3B). In the last method, barriers preventing immersion of hybridization solution are constructed by printing a single substance or substance mixture around the arrays (Fig. 3C) . For example, solid pins for the gridder can be printed as a barrier. Alternatively, a hydrophobic material can be printed to prevent the outflow of buffer between arrays. As described above, multiple hybridization reactions can be independently conducted on a single surface layer by printing framed barriers on the platform surface.

Then, in the present invention, cDNAs hybridized to the nucleotide probes immobilized on the platform are detected to measure the expression level of the cluster genes in the disease cell treated with the test compound (step (c)).

According to the present invention, when a cDNA derived from a gene constituting a cluster is present in the cDNA sample, the cDNA hybridizes with a nucleotide probe immobilized on the platform. Thus, the expression level of the cluster gene can be measured by detecting the hybridized cDNA. One skilled in the art can appropriately detect the hybridized cDNA depending on the kind of substance used for the labeling of the cDNA sample. For example, when the cDNA is labeled with a fluoresce substance, the cDNA can be detected by monitoring the fluorescence signal with a scanner.

Next, in the present invention, the expression levels of the cluster genes in the disease cell treated with the test compound are determined to see if they are restored to the levels found in the normal cells (step (d)).

In a preferred embodiment of this invention, the expression levels of the cluster genes in the disease cells and control cells are measured simultaneously. Herein, the term "control cells" refers to cells suitable for estimating the restoration of the expression level of a cluster gene in the disease cell to a normal level following treatment with a test compound. The control cells are preferably normal cells. According to the method of this invention, the expression levels of a cluster gene in two kinds of cells can be measured at once by labeling cDNA samples prepared from the two kinds of cells with different fluorescent agents. For example, one of the above cDNA samples may be labeled with the fluorescent agent Cy5 and the other with Cy3. The relative intensity of each fluorescent signal indicates the relative value corresponding to the expression levels of the cluster genes in the disease cells and control cells, respectively (Duggan et al., Nat. Genet. 21: 10-14, (1999)). When the measured expression levels in the disease cells and control cells are substantially the same, the expression levels of the cluster genes in the disease cells treated with the test compound are judged to be restored to that found in the normal cell. In this case, the compound used for the treatment of the disease cells is expected to have a therapeutic effect on diseases characterized by the cluster genes whose expression levels were restored by the compound.

In a preferred embodiment of the present invention, the diseases on which a compound is expected to be effective can be specified: using a platform immobilized with nucleotide probes that hybridize with cluster genes corresponding to various kinds of diseases, respectively, and estimating the effect of a test compound on the diseases. The disease gene, functionally located upstream to the cluster gene whose expression level is restored to the normal levels, is predicted to be a target of the compound.

Furthermore, this invention provides a method of screening for candidate compounds to treat a specific disease by utilizing the above estimation method. In the screening method, following the above steps of (a) to (d), the test compound is selected which restores the expression level of the gene constituting the cluster of the disease cell treated with the compound to that in the control cell (step (e)).

The method of this invention enables high-throughput screening of candidate compounds for treating a specific disease. Such compounds obtained by the screening of this invention are expected to exert a biological effect in natural cells compared to compounds obtained by a screening with an artificial reporter. A compound isolated by the screening method of this invention is also included in the present invention.

### Brief Description of the Drawings

Fig. 1 is a photograph showing the result of a two-dimensional clustering analysis of the multiple genes in multiple tumor samples. Red color (mainly found in regions consisting of the type B/cluster 1 and the type A/cluster 2) indicates the genes whose expression levels were higher than the median among all the samples . Green color (mainly found in regions consisting of the type A/cluster 1 and the type B/cluster 2) indicates the genes whose expression levels were the median or lower. The color intensity (darkness/lightness) correlates with the ratio between the expression level of the test (tumor) sample and the control sample.

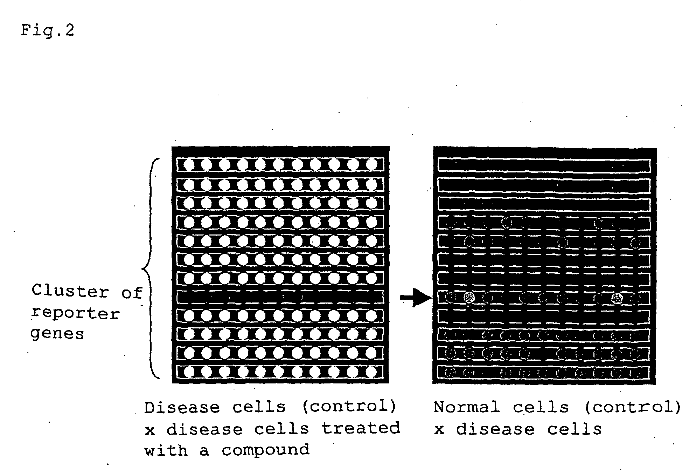

Fig. 2 depicts photographs showing the result of a high-throughput screening by microarrays. The eleven genes in the respective horizontal rows constitute a single gene cluster identified for a specific disease. Mainly 11 genes in the 4th, 5th, 8th, .10th, 11th, and 12th rows from the top of the right panel are red. On the other hand, mainly 11 genes in the 8th row of the left panel, and the 1st, 2nd, and 3rd rows of the right panel are green.
Fig. 3 depicts schematic illustrations of three kinds of constructible platforms with barriers between arrays. (A) shows channels separating each array. (B) shows high-built seals. (C) shows another type of barrier with a solid pin or printing of a hydrophobic substance.
Fig. 4 shows a figure of a slide containing.48 physically separated areas for analyzing 48 different test samples. Each area is printed with a single pin.

### Best Mode for Carrying out the Invention

The present invention will be described in detail below with reference to Examples, but it is not to be construed as being limited thereto.

### [Example 1] Two-dimensional clustering analysis

Two-dimensional clustering analysis of multiple genes of multiple tumor samples was performed. Fig. 1 shows the virtual result of screening of 38 different tumor samples against a panel containing 16 different genes. All the tumor samples were analyzed with respect to a common control sample.

In Fig. 1, both the tumor samples and the genes are ordered into groups or clusters by the two-dimensional clustering. The 16 genes were separated into two clusters consisting of eight genes where each cluster was a group of genes whose expression patterns were similar among all the test samples. Similarly, the 38 tumor .samples were grouped into two clusters of 19 samples where each cluster consisted of tumor samples wherein the analyzed genes exhibited a similar expression pattern. By referring and comparing the functions of known genes classified in the same cluster, other genes in the cluster are often revealed to have a related function to the known gene. For example, according to a result obtained by monitoring the expression changes of 8613 genes in fibroblasts in response to serum addition in a time-course revealed that the genes can be categorized into 10 different clusters (Iyer VR, Eisen MB, Ross DT, Schuler G, Moore T, Lee JC, Trent JM, Staudt LM, Hudson J Jr, Boguski MS, Lashkari D, Shalon D, Botstein D, Brown PO., The transcriptional program in the response of human fibroblasts to serum. Science 283 (5398):83-87 (1999)). These clusters comprise genes participating in specific cellular processes, such as tissue regeneration, cell wall maintenance, and biogenesis of specific compounds.

### [Example 2] High-throughput screening (HTS) microarray

A disease cell line was treated with a compound and the response of a cluster was used as the indicator for screening. Specifically, HTS microarray analysis was carried out to identify compounds that restore the gene expression level of the cluster to a normal level. In this assay, a diseased tissue without treatment was compared as a control to the diseased tissue treated with a compound.

The result is shown in Fig. 2. Eleven genes shown in each horizontal row constitute a single cluster identified with respect to a specific disease. A compound that reduces the expression levels of the cluster genes, whose expression levels are increased in the diseased tissue (the left array) compared to the normal tissue (the right array) , stands as a candidate for which a more detailed assay is to be carried out. Although the target of the compound it self cannot be directly identified by this method, the target can be presumed to be located within the influenced cluster or further upstream of the cluster.

### Industrial Applicability

The present invention provides a method of high-throughput screening for candidate compounds for the treatment of a disease where the compounds have a biological effect *in vivo.* According to the method of this invention, the expression of genes inherently present in cells are used as an indicator for screening candidate compounds for the treatment. Therefore, the result directly reflects the effect of a test compound on the cell. Thus, the compound obtained by the method of this invention is expected to work effectively as a therapeutic agent for the disease. Furthermore, the method of this invention allows simultaneous screening of candidate compounds for the treatment of various diseases in a single analysis.

## Claims

1. A method for estimating whether a test compound can be a candidate for treating a specific disease, which method comprises the steps of:
(a) providing
(i) cDNAs derived from a disease cell treated with a test compound, and
(ii) a platform immobilized with nucleotide probes that respectively hybridize with multiple cluster genes constituting a cluster related to the disease,
(b) contacting the cDNAs of (a) (i) with the platform of (a) (ii),
(c) measuring the expression levels of the cluster genes in the disease cell treated with the test compound by detecting cDNAs that hybridized with the nucleotide probes immobilized on the platform, and
(d) estimating whether the expression levels of the cluster genes in the disease cell treated with the test compound are restored to that in normal cell ;

2. A method of screening for candidate compounds for the treatment of a specific disease, which method comprises the steps of:
(a) providing
(i) cDNAs derived from a disease cell treated with a test compound, and
(ii) a platform immobilized with nucleotide probes that respectively hybridize with multiple cluster genes constituting a cluster related to the disease,
(b) contacting the cDNAs of (a) (i) with the platform of (a) ( ii) ,
(c) measuring the expression levels of the genes constituting the cluster in the disease cell treated with the test compound by detecting cDNAs that hybridized with the nucleotide probes immobilized on the platform,
(d) estimating whether the expression levels of the cluster genes in the disease cell treated with the test compound are restored to that in normal cell, and
(e) selecting the test compound that restored the expression levels of the genes constituting the cluster in the disease cell treated with the compound to that in control cell; and,

3. A candidate compound for the treatment of a specific disease isolated by the method according to claim 2.
